# EUROPEAN PATENT APPLICATION

(11) **EP 2 871 245 A1**
(43) Date of publication of application: **13.05.2015**
(21) Application number: 13192134.8
(22) Date of filing: 08.11.2013
(51) Int. Cl.: C12R 1/125, A01N 63/00, A01N 63/02, C07K 7/56

(54) **A bacillus subtilis strain deposited under deposit number CECT 8258 and method for protecting or treating plants**

(71) Applicant: Symborg, S.L., 30100 Murcia (ES)
(72) Inventor: Fernández Martín, Félix, 30100 Murcia (ES); Vicente Sánchez, Javier, 30100 Murcia (ES); Bernabé García, Antonio José, 30100 Murcia (ES); Torrecillas Sánchez, Alejandro, 30100 Murcia (ES)
(74) Representative: Carvajal y Urquijo, Isabel

(57) **Abstract**

*A Bacillus subtilis* strain deposited under deposit number CECT 8258 and a method for protecting or treating plants, plant parts, plant roots or plant seeds from fungal and bacterial infestations, comprising applying a composition comprising the *Bacillus subtilis* strain deposited under deposit number CECT 8258 or a supernatant obtained from a culture of the *Bacillus subtilis* strain deposited under deposited number CECT 8258 or an extract isolated from *Bacillus subtilis* strain deposited under deposited number CECT 8258 to said plants, plant parts, plant roots or plant seeds. Two novel compounds are also disclosed, of formula 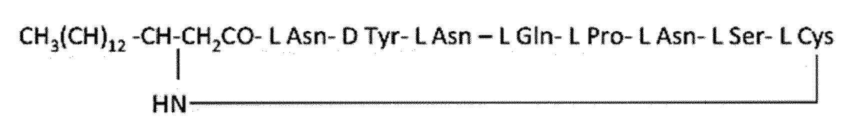 and formula

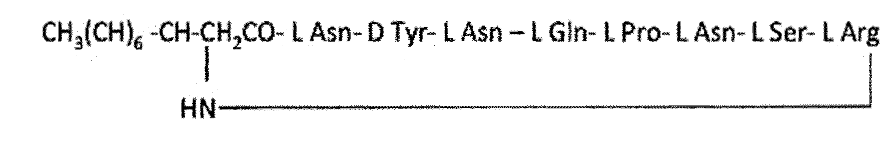

## Description

### FIELD OF THE INVENTION

The present invention is in the field of biopesticides. More specifically, this invention relates to a novel strain of *Bacillus subtilis* CECT 8258 that inhibits a range of difficult to control fungal and bacterial plant diseases. The invention also relates to fungicidal and bactericidal compositions comprising this novel *Bacillus* strain and the metabolites, both alone and in combinations, produced by this strain, either alone, or in combination with other chemical and biological pesticides.

### BACKGROUND OF THE INVENTION

For some time, it has been known that certain microorganisms possess biological activity that is useful in the control of plant diseases. While process has been made in identifying and developing biological pesticides, the majority of pesticides in use continue to be synthetic compounds. Many of these have unwanted side effects on non-target organisms, the environment, the users of these pesticides, as well as resulting in pesticide residues in food that many consumers would prefer to avoid.

Biological control offers an attractive alternative to synthetic chemical fungicides. Biopesticides can be safer to the environment and users, degrade more quickly, and will not result in chemical residues on treated crops. Biopesticides developed from microorganisms are extremely useful for integrated pest management programs and as tools to combat the development of pest resistance to synthetic chemicals. In addition, biopesticides can be developed in a shorter time period at less cost than synthetic pesticides.

For some time, it has been known that some bacteria in culture media under favorable conditions produce phytotoxic metabolites such as lactic and acetic acid, cyanide, and antibiotics. The metabolite produced depends upon the environmental conditions in which the bacteria are located. Therefore, secondary metabolites produced by bacteria are dependent upon the environment and the availability of specific enzyme complex.

Secondary metabolites are byproducts of primary metabolites and although not necessarily essential for the bacteria, may generate some advantages to ensure their survival. Bacterial have their secondary metabolite synthesis influenced by environmental conditions. Screening programs have identified *Bacillus* spp. strains that exhibit antifungal activity. These strains have been shown to produce zwittermicin-A and/or kanosamine, two antibiotic compounds that are effective against the soil born disease damping off, caused by *Phytophthora* spp. or *Sclerotinia minor.*

U.S. Patent Number 5,049,379 to Handelsman et al. describes how zwittermicin-A reduces damping off in alfalfa and soybeans. Coating the seed with *B. cereus* ATCC 53522 resulted in reduced infection by the damping off fungus. In addition, applying a spore based formulation derived from *B. cereus* strains to either soybean seeds or the soil surrounding them has been demonstrated to improve soybean yield in field studies. Methods of applying biopesticides are well known in the art and include wettable powders, dry flowables, microencapsulation of the active agents, liquid or solid formulations of the antibiotic fractions from cultures (See e.g., U.S. Patent Number 5,061,495 to Rossall or U.S. Patent Number 5,049,379 to Handelsman).

*Bacillus subtilis* is an antagonistic bacteria that acts through production of antibiotics as well as by parasitism and competition for space and nutrients. Microorganisms acting through antibiotics generally have a broad spectrum of action, particularly fungal inhibition. This approach is more efficient than other antifungal mechanisms of action.

It has been disclosed that the activity of the soil fungus, *Pythium aphanidermatum,* that causes cottony cucumber leak, can be suppressed using zwittermicin producing *B. cereus* strain UW85. The production of anti*-Botrytis* and anti*-Alternaria* antibiotics by two *Bacillus* strains, *B. subtilis* CL27 and *B. pumilus* CL 45 has been reported. In vitro testing demonstrated both the whole broth and cell-free filtrates were active against Botrytis and *Alternaria.* U.S. Patent Number 5,597,565 to Leifert et al. discloses that *B. subtilis, B. pumilus,* and *B. polymyxa* are effective at inhibiting post harvest disease causing fungi. They also disclose, but do not identify, the presence of antibiotics produced in the cell-free culture filtrate and their activity.

U.S. Patent Number 5,344,647 to Rossall discloses *B. subtilis* strains with broad anti-fungal activity. Numerous researchers in the art have disclosed the use of *Bacillus* spp. and *Bacillus subtilis,* specifically, as biocontrol agents of fungal plant pathogens, including the use for control of post harvest fruit rots. U.S. Patent Number 5,047,239 to Pusey discloses the use of *B. subtilis* B-3 to inhibit brown rot, grey mold, and bitter rot in peaches (pre harvest), apples (postharvest), and grapes (postharvest). U.S. Patent Number 5,780,080 to Leifert et al. discloses use of *B. subtilis* NCIMB 40491 to inhibit the growth of organisms of the postharvest disease causing fungi *Botrytis cinerea* and *Alternaria brassicicola* in cabbages. U.S. Patent Number 6,203,228 to Heins et al. discloses *B. subtilis* AQ713 that exhibits insecticidal, antifungal and antibacterial activity. They also discuss the antibacterial and antifungal compounds produced by this strain. U.S. Patent Number 6,245,551 to Lehman et al. discloses a novel *Bacillus pumilus* strain which exhibits antifungal activity, but no antibacterial activity.

U.S. Patent Number 5,403,583 to Liu et al. disclose a *Bacillus megaterium* ATCC 55000 and a method to control the fungal plant pathogen *Rhizoctonia solani.* In vitro antagonism of *B. megaterium* against *Drechslera oryzae, Alternaria alternata,* and *Fusarium roseum* has been disclosed. The most active of the components in the culture filtrate was an antibiotic highlight soluble in water and methanol which proved to be a polyoxin-like lipopeptide.

*Bacillus* spp. have been known for some time to produce antifungal and antibacterial secondary metabolites. The fungicidal compound zwittermicin-A and the compound kanosamine produced by *Bacillus* sp. have been identified in the prior art.

Another group of *Bacillus* metabolites are the cyclic lipopeptides of the iturin class, some of which are potent fungicidal agents. The mode of action of these lipopeptides has been reported to be due to interactions with fungal membranes, creating transmembrane channels that permit the release of vital ions.

The structure/activity relationship of the iturins by synthesizing a number of analogues differing in the amino acid chain length has been studied. The activity of the iturins increased with the length of the fatty acid side chain and terminal branching (Bland et al., "Iturin-A, an antifungal peptide produced by Bacillus subtilis" Proc. Plant Growth Regulation Soc. Am. 22:102-107 (1995)).

Another group of lipopeptides isolated from B. *cereus* are the plipastatins. These compounds were originally identified in a study of inhibitors of porcine pancreatic phospholipase A2, but later work found they also inhibit some plant pathogenic fungi including *Botrytis, Pyricularia,* and *Alternaria* (Yamada et al., "Biological activity of antifungal substances produced by Bacillus subtilis" J. Pesticide Sci. 15(1):95-96 (1990)). Yamada also reported synergistic effects observed between iturin A and the plipastatins, both produced by the same *B. subtilis* strain.

Another cyclic lipopeptide produced by *B. subtilis* is surfactin, which possesses exceptional surfactant activity. The novel microorganism *B. subtilis* AQ713 produces A iturins, plipastatins, and surfactins. Production of this combination of lipopeptides is key to its usefulness as a biocontrol agent.

*Sclerotinia sclerotiorum* is an important phytopathogenic soil fungus that causes economic losses in lettuce potatoes, cereals, and a wide number of other important crops. Signs and symptoms caused by *S. sclerotiorum* vary depending on the host. However, the most obvious sign is the appearance of white fluffy mycelia growth that will later produce sclerotia. The fungus infects and produces mycelium at the base of the plant that will eventually move up the stem causing it to rot. Sclerotia are a compact mass of hardened fungal Código de campo cambiado mycelium containing food reserves. One role of sclerotia is to survive environmental extremes, making control of this disease challenging.

In the absence of control, *Sclerotinia* can cause losses of up to 60 percent under conditions favorable to the disease. Control and eradication of the pathogen is difficult as sclerotia can survive in soil for years in the absence of any host or conditions favorable for development. Primary chemical control methods have included soil fumigation, but these compounds (e.g. methyl bromide) are under extreme regulatory pressure due to high toxicity and potential adverse effects to environment and non-target organisms. It has been reported that even with the use of effective control methods, there may still be sufficient sclerotia to cause disease. Studies to evaluate the potential of *Bacillus* spp. for *Sclerotinia* have shown the results are highly dependent upon the root exudates produced by the plant host.

### DESCRIPTION OF THE INVENTION

The term "bacteria" or "bacterium" includes any prokaryotic organism that does not have a distinct nucleus.

The term "fungus" or "fungi" includes a wide variety of nucleated spore-bearing organisms that are devoid of chlorophyll. Examples include yeast, molds, mildews, rusts, and mushrooms.

"Fungicidal" means the ability of a substance to increase mortality or inhibit the growth rate of fungi.

"Antibiotic" includes any substance that is able to kill or inhibit a microorganism. Antibiotics may be produced by a microorganism or by a synthetic process or semisynthetic process. The term includes substances that inhibit or kill fungi, such as zwittermicin-A or kanosamine.

"Antifungal" includes any substance that is able to kill or inhibit the growth of fungi.

"Antibacterial" includes any substance that is able to kill or inhibit the growth of bacteria.

The term "culturing" refers to the propagation of organisms on or in media of various kinds.

"Whole broth culture" refers to a liquid culture containing both cells and media. "Supernatant" refers to the liquid broth remaining when cells grown in the broth are removed by centrifugation, filtration, sedimentation, or other means well known in the art.

The term "solvent" includes any liquid that holds another substance in solution. "Solvent extractable" refers to any compound that dissolves in a solvent and which then may be isolated from the solvent.

A preferred embodiment of the invention is a *Bacillus subtilis* strain deposited under deposit number CECT 8258, herewith strain of the invention.

The strain of *Bacillus subtilis* of the invention was deposited on 18 December, 2012 on the Colección Española de Cultivos Tipo (CECT), sited at Parc Científic Universitat de València, Catedrático Agustín Escardino, 9, 46980 Paterna (Valencia, Spain) by the depositor Symborg, S.L., sited at Edificio Ceeim, Campus Universitario, S/N, 30100 Murcia (Spain).

The strain of *Bacillus subtilis* was identified by the depositor with the reference SB0003, and received the CECT accesion number CECT 8258 after the International Depositary Authority declared the strain as viable.

The strain was identified based on the utilization of molecular characterization, physiological and proteomic study. Microscopic observation identified the strain is formed by Gram-positive bacilli, capable of sporulation, therefore belonging to the genus *Bacillus.* The sample was seeded using various liquid and solid media, where growth was found after incubation at between 30 and 37 °C. Isolated colonies of the strain were then subjected to biochemical tests, the results of which are listed in the table below.

A more extensively characterization of CECT 8258 was conducted by CECT.

Isolated submitted as: *Bacillus* sp. Strain SB0003.

Isolate Identification: Using API strips, specifically API 50CHE, following the manufacturer's instructions (Biomerieux), which advises use of the API 20E gallery to complement the API 50CH gallery, and incubation at 37 °C. Readings were conducted following 24 and 48 hours incubation at the optimal growth temperature of the strain under aerobic conditions.

Cellular morphology: The motile cells are found singly, with two endospore formed on the subterminal region. The cells are uniformly stained Gram positive.

Colony morphology: The colonies are opaque and entire with low convex elevation, glistening and smooth appearance. The margins are white and regular.

**Table 1. API 50CHE gallery Results.**

| | | | |
|---|---|---|---|
| Rods | + | Colony entire | + |
| Rods straight | + | Colony erose | - |
| Rods curved | - | Colony lobate | - |
| Cell singles | + | Colony circular | - |
| Ends tarpered | - | Colony irregular | - |
| Ends rounded | + | Colony rhizoid | - |
| Ends squared | - | Colony low convex | + |
| Endospore formed | + | Colony high convex | - |
| Sporanggium swollen | - | Colony flat | - |
| One spore/cell | - | Colony raised | - |
| Spore round | - | Colony glistening | + |
| Spore cylindrical | + | Colony dull | - |
| Spore oval | - | Colony dry | - |
| Spore central | - | Colony smooth | + |
| Spore terminal | - | Colony rough | - |
| Spore subterminal | + | Catalase | + |
| Gram positive | + | Insoluble Brown pigment | - |
| Gram negative | - | Insoluble Black pigment | - |
| Gram variable | - | Insoluble yellow pigment | - |
| Gram stained | + | Insoluble orange pigment | - |
| Vacuoles present | - | Insoluble red pigment | - |
| Colony translucent | - | Soluble Brown pigment | - |
| Colony Transparent | - | Soluble Black pigment | - |
| Cells motile | + | Soluble yellow pigment | - |
| Growth at 150 C | + | Oxidase | + |
| Growth at 260 C | + | Aerobe | + |
| Growth at 300 C | + | Facultative | - |
| Growth at 370 C | + | Microaerophile | + |
| Growth at 450 C | + | Anaerobe | - |
| Growth at 500 C | + | Growth at pH 4.5 | + |
| Growth at 550 C | - | Growth at pH 6.5 | + |
| Growth at 550 C | - | Growth at pH 9 | + |
| Growth at 600 C | - | Growth at 2 % NaCl | + |
| Starch Hidrolysis | + | Growth at 5 % NaCl | + |
| Colony opaque | + | Growth at 10 % NaCl | + |

**Table 2. API 20E gallery Results.**

| Tube | Substrate | Enzyme Function | 24 h | 48h |
|---|---|---|---|---|
| ONPG | 2-nitro-fenil-βD galactopiranoside | β-galactosidase | + | + |
| ADH | L-arginine | Arginine dihidrolase | - | - |
| LDC | L-lysine | Lysine decarboxylase | - | - |
| ODC | L-ornithine | Ornithine decarboxylase | - | - |
| CIT | Trisodium Citrate | Citrate utilization | d | + |
| H2S | Sodium thiosulfate | H₂S Production | - | - |
| URE | Urea | Urease | - | - |
| TDA | L- Tryptophane | Tryptophane deaminase | - | - |
| IND | L- Tryptophane | Indole Production | - | - |
| VP | Sodium piruvate | Acetoine Production | - | + |
| GEL | Bovine gelatine | Gelatinase | + | + |
| GLU | D-Glucose | Oxidation/Fermentation | + | - |

Additional analysis of proteins was determined following the protocol recommended by Bruker Daltroniks by the extraction of ethanol/formic acid. Spectrograms showed a close correlation between this strain and known *B. subtilis* strains.

Finally, genetic sequence of *Bacillus subtilis* strain CECT 8258 was completed, and found to match to greater than 99% similarity, with more than 40 different *B. subtilis* strains. Based upon this analysis, it was concluded that strain CECT 8258 is a member of *Bacillus subtilis.* In addition, the peptide fingerprint of strain CECT 8258 through 2D electrophoresis differs from other species with regard to the protein content present in the NCBI databank.

Based on all available physiological and biochemical data, the strain CECT 8258 most closely resembles *Bacillus subtilis.*

In another aspect, the invention encompasses a method for protecting or treating plants, plant parts, plant roots or plant seeds from fungal and bacterial infestations, comprising applying
- a composition comprising the *Bacillus subtilis* strain deposited under deposit number CECT 8258 or
- a supernatant obtained from a culture of the *Bacillus subtilis* strain deposited under deposited number CECT 8258
to said plants, plant parts, plant roots or plant seeds,
herewith method of the invention.

A particular embodiment is the method of the invention, wherein said fungal infestations are caused by a member of the Sclerotineaceae family.

A particular embodiment is the method of the invention, wherein said fungal infestations are caused by fungi selected from the group composed by Erwinia amylovora, Macrophomina phaseolina and Botrytis cinerea.

A particular embodiment is the method of the invention, wherein said fungal infestations are caused by foliar fungal pathogens which attack the plant foliage.

A particular embodiment is the method of the invention, wherein said fungal infestations are caused by soil fungal pathogens which attack plant roots.

The strain of the invention can be combined with other known pesticides. Thus, a particular embodiment is the method of the invention, wherein a biological or chemical pesticide is further applied to said plants, plant parts, plant roots or plant seeds.

A particular embodiment is the method of the invention, wherein the *Bacillus subtilis* strain deposited under deposit number CECT 8258 is applied as a whole broth culture.

A particular embodiment is the method of the invention, wherein the *Bacillus subtilis* strain deposited under deposited number CECT 8258 is applied to the soil surrounding the plant roots.

A particular embodiment is the method of the invention, wherein the *Bacillus subtilis* strain deposited under deposit number CECT 8258 is applied as wettable powders, granules, aqueous suspensions, emulsifiable concentrates or microencapsulations.

The strain of the invention can be formulated as wettable powders, granules, and other similar formulations or can be microencapsulated in a suitable medium or similar formulations. Examples of formulations include, but are not limited to, soluble powders, wettable granules, dry flowables, aqueous flowables, wettable dispersible granules, emulsifiable concentrates, and aqueous suspensions. Other suitable formulations will be known to those skilled in the art.

A particular embodiment is a compound of formula

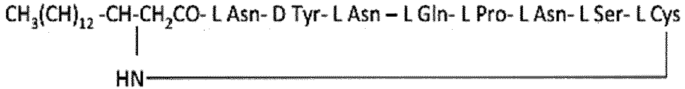

A particular embodiment is a method for protecting or treating plants, plant parts, plant roots or plant seeds from fungal and bacterial infestations, comprising applying the compound of formula 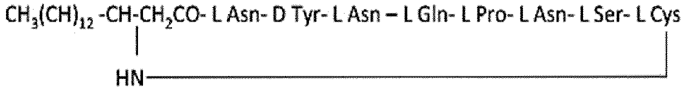 to said plants, plant parts, plant roots or plant seeds.

Particularly, said fungal infestations are caused by a member of the *Sclerotineaceae* family. More particularly, said fungal infestations are caused by fungi selected from the group composed by *Erwinia amylovora, Macrophomina phaseolina* and *Botrytis cinerea.*

A particular embodiment is a compound of formula

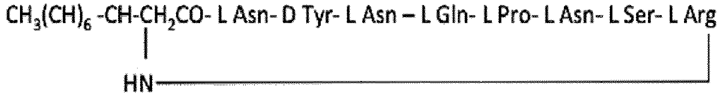

A particular embodiment is a method for protecting or treating plants, plant parts, plant roots or plant seeds from fungal and bacterial infestations, comprising applying the compound of formula 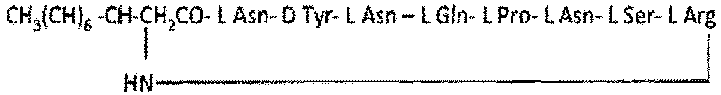 to said plants, plant parts, plant roots or plant seeds.

Particularly, said fungal infestations are caused by a member of the *Sclerotineaceae* family. More particularly, said fungal infestations are caused by fungi selected from the group composed by *Erwinia amylovora, Macrophomina phaseolina* and *Botrytis cinerea.*

### BRIEF DESCRIPTION OF THE FIGURES

Figure 1. Rate Impact on lettuce plants treated and untreated with 70 days Biofungicide to curative treatment.
Figure 2. Rate Impact on lettuce plants treated and untreated with 70 days Biofungicide to preventive treatment.
Figure 3. Effect of *Bacillus subtilis* strain CECT 8258 on *Erwinia amylovora* disease development in pears following foliar and soil treatment.
Figure 4. Mass spectra of *Bacillus subtilis* strain CECT 8258 metabolites (800 - 1300 m/z) representing Kurstakins, Surfactins and Iturins. The peaks at m/z = 1199.2 and 1213.4 corresponds to the new compounds discloses in Example 4.
Figure 5. Mass spectra of *Bacillus subtilis* strain CECT 8258 metabolites (1300 - 1800 m/z) representing Fengycins, Polymixins and Bacitraxins.

### EXAMPLES OF THE INVENTION

### Example 1. In vitro activity of Bacillus subtilis CECT 8258 against Sclerotinia sclerotiorum and Macrophomina phaseolina

A bacterial suspension of the strain CECT 8258 in a culture medium was developed and calibrated by a spectrophotometer (A = 1.002 at λ = 623) for 24 and 48 hours of culture. With the aim of evaluating the metabolites excreted by the bacterial culture medium, broths from selected times were autoclaved for 40 minutes at 120 ° C, thereby ensuring complete elimination of microbial vegetative cells and spores.

In order to evaluate the inhibitory activity of the metabolites against *Sclerotinia sclerotiorum* and *Macrophomina phaseolina* four concentrations of metabolites in the media were selected (0% (control), 5%, 15% and 20%) at 24 and 48 hours of culture in potato dextrose agar medium (PDA). Mycelial discs (5 mm diameter) were placed at the center of the plate of *Sclerotinia sclerotiorum* and *Macrophomina phaseolina,* and were incubated in a cold chamber at 20 ° C for Sclerotinia *sclerotiorum* and 30 ° C for *Macrophomina phaseolina.* The mycelial growth was evaluated at 24, 48, and 72 hours of incubation.

**Table 3. Biological activity of the metabolites of the strains CECT 8258 on the development of fungal colonization of Sclerotinia sclerotiorum on Petri dishes.**

| Metabolites from 24 hours of fermentation | | | |
|---|---|---|---|
| Treatments | Diameter (24 h) mm | Diameter (48 h) mm | Diameter (72 h) mm |
| Control | 7,5 a | 17,75 a | 27.8 a |
| 5% | 7,6 a | 15,5 b | 20.3 b |
| 15% | 5,41 b | 13,75 b | 18.6 c |
| 20% | 5 b | 11,25 c | 12.3 d |
| Es x | 0.2 * | 0.3** | 0.5* |

| Metabolites from 48 hours of fermentation | | | |
|---|---|---|---|
| Treatments | Diameter (24 h) mm | Diameter (48 h) mm | Diameter (72 h) mm |
| Control | 7,5 a | 17,75 a | 30.4 a |
| 5% | 5 b | 15,5 b | 18.6 b |
| 15% | 5 b | 9,25 c | 11.2 c |
| 20% | 5 b | 9,25 c | 10.4 c |
| Es x | 0.3** | 0.4* | 0.4** |

**Table 4. Biological activity of the metabolites of the strains CECT 8258 on the development of fungal colonization of Macrophomina phaseolina on Petri dishes.**

| Metabolites from 24 hours of fermentation | | | |
|---|---|---|---|
| Treatments | Diameter (24 h) mm | Diameter (48 h) mm | Diameter (72 h) mm |
| Control | 11,25 a | 24,5 a | 33.6 a |
| 5% | 7,7 b | 20 b | 21.3 b |
| 15% | 7,625 b | 21 b | 23 b |
| 20% | 8 b | 16,25 c | 19.5 c |
| Es x | 0.45* | 0.5* | 0.9** |

| Metabolites from 48 hours of fermentation | | | |
|---|---|---|---|
| Treatments | Diameter (24 h) mm | Diameter (48 h) mm | Diameter (72 h) mm |
| Control | 11,875 a | 24 a | 38 a |
| 5% | 6,875 b | 10,75 b | 14 b |
| 15% | 5,875 b | 7,25 bc | 8 c |
| 20% | 5 b | 5 c | 8 c |
| Es x | 0.3** | 0.4* | 0.35** |

Significant differences in development of fungal colonies were observed with both species. As dose of metabolites increased, the inhibition of fungal development increased. Metabolites from culture medium incubated for 48 hours produced greater inhibition in both species.

### Example 2. Activity in vivo of Bacillus subtilis CECT 8258 against Sclerotinia sclerotiorum in lettuce

The plant species studied was *Lactuca sativa* L "Iceberg" type, cropping cycle 90 days. The transplant was performed by planting in containers (pots) of 75 cm x 15 cm x 15 cm in size, with three seedlings/pot, containing a clay soil favorably to infection with *Sclerotinia sclerotiorum.*

Two conditions were studied to evaluate disease control, a condition of either preventive or curative application timing. Both conditions evaluated three doses of application and untreated control treatment, for a total of 8 treatments. Here are the variants studied.
T1: Control untreated
T2: 3 l / ha
T3: 4 l / ha4
T4:5 l / ha

Finally, in the case of preventive condition, in treatments where Biofungicide is used, a dose of 2 l / ha has been applied, applied 20 days after the first dose and for the curative condition, two doses of 2 l / ha has been applied every 3rd day after the first inoculation.

Foliar fresh and dry weight (FFW, FDW) and root fresh and dry weight (RFW, RDW) were evaluated after 90 days culture. Ten plants per treatment and were separated into different organs and weighed on a Sartorius balance model. To obtain dry weight, the samples were placed in an oven at 80 ° C to constant weight. The ratios between foliar and dry biomass were calculated.

Percent mortality and disease involvement were calculated and rhizosphere soil samples from each plant and treatment, serial dilutions to determine the most probable number of microorganisms, the concentrations of bacteria and fungi rhizosphere. For bacterial growth medium was used and Nutrient Agar for fungi Rose Bengal.

**Table 5. Foliar Fresh Weight (FFW) g, Foliar Dry Weight (FDW) g of lettuce plants treated and untreated with Biofungicide to cure 70 days.**

| Cure | FFW | FDW |
|---|---|---|
| Control | 177,26±20,38 | 71,4±23,04 |
| 3 liters/Ha. | 399,4±17,21 | 71,7±8,97 |
| 4 liters/Ha. | 354,88±30,00 | 100,8±13,89 |
| 5 liters/Ha. | 266,2±19,20 | 37,6±10,92 |

**Table 6. Foliar Fresh Weight (FFW) g, Foliar Dry Weight (FDW) g of lettuce plants treated and untreated with Biofungicide to preventive 70 days.**

| Preventive | FFW | FDW |
|---|---|---|
| Control | 193,57±13,58 | 69,83±14,01 |
| 3 liters/Ha. | 312,93±9,50 | 135,28±15,57 |
| 4 liters/Ha. | 321,89±12,09 | 119,55±8,81 |
| 5 liters/Ha. | 309,29±18,82 | 97,31±11,84 |

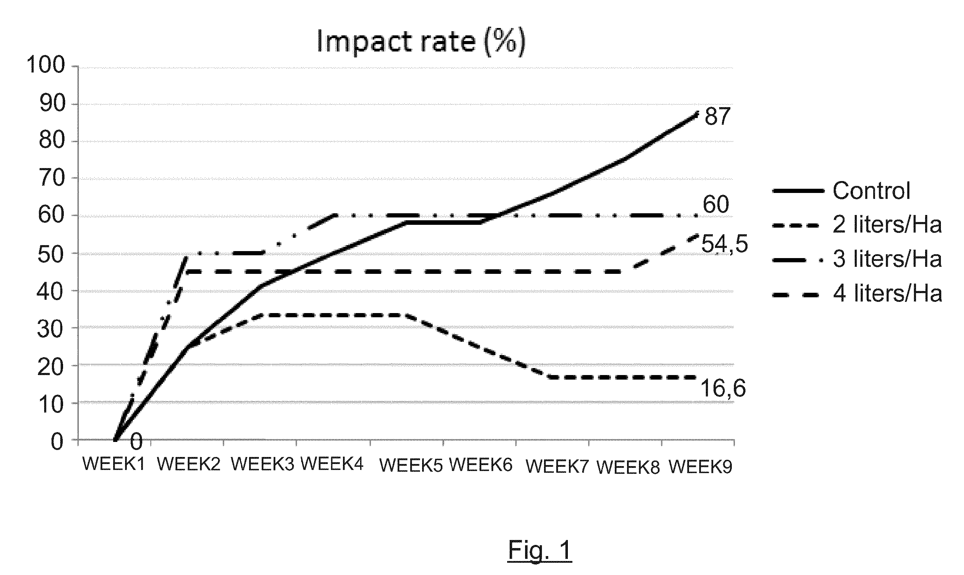

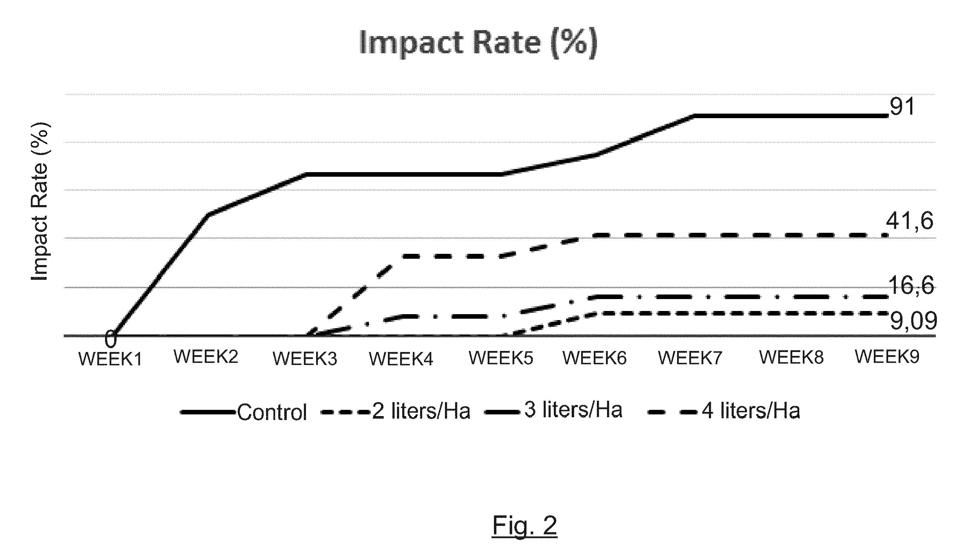

All three rates of *B. subtilis* CECT 8258 reduced the disease infection when applied as a curative treatment (Figure 1). The 3 L/ha rate resulted in best protection with <20% infection at the end of the study compared to the untreated control with >80% infection. When applied as a preventative treatment, all three rates again reduced disease with the 3 L/ha and 4 L/ha treatments both providing excellent protection (<20% infection) compared to the untreated control with 90% infection (Figure 2).

### Example 3. Activity in vivo of Bacillus subtilis CECT 8258 against Erwinia amylovora in pears.

A total of five trials were conducted in the field using three varieties of pears - Ercolini, Castell, and Etruscan - to evaluate the performance of *Bacillus subtilis* strain CECT 8258 against the bacterial disease fire blight caused by *Erwinia amylovora.* At all trial sites, applications were made when trees were at 30% bloom and the disease was already present in the trees. A minimum of 80 trees per treatment were used in each trial. An initial count of infection sites was made at the time of treatment.

The treatments used in the studies were:
1. Untreated plants
2. Foliar treatment with 3 liters / ha.
3. Soil and foliar treatment, both at 3 liters / ha

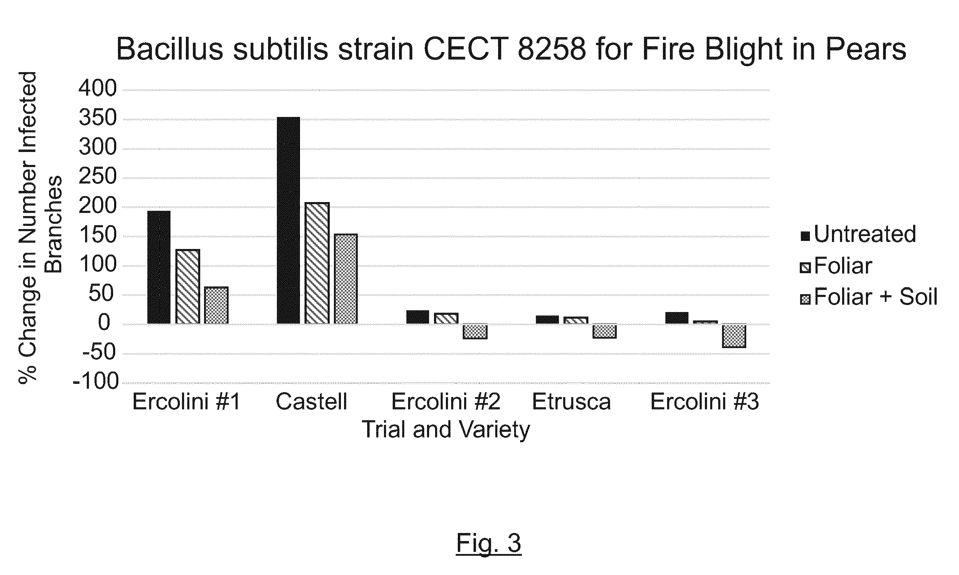

After application, the number of infected branches was counted. The percent change in the number of branches infected with *Erwinia amylovora* was calculated and compared to the untreated. The results show that with all three varieties, the number of diseased branches was reduced by at least 50% in comparison to the control. This trend was observed both under low disease pressure and under very high disease pressure. The trees receiving both soil and foliar treatment showed the greatest reduction in percent increase in *Erwinia amylovora* infection. In some cases where disease pressure was lower, the trees receiving both soil and foliar treatment had a reduction in percent infection, indicating a curative activity. A comparison of the percent change in infected branches for the three treatments can be seen in Figure 3.

### Example 4. Separation, Analysis and Activity of the Compounds Present in CECT 8258

Initially, the complete lipopeptides fraction were extracted from the bacterial supernatant and its mass spectra were obtained using an Agilent TOF 6000 Series Mass Spectrometer (Agilent Technologies, Santa Clara, CA, USA), equipped with an AP-MALDI Ion Source with a N₂ laser (337 nm). Mass spectra were acquired by accumulating 200 laser shots in reflectron mode in positive mode in the range from 100-2200 m/z. The main TOF-MS parameters were as follows: capillary temperature, 350 °C; capillary voltage, 3500 V; fragmentor voltage, 215 V; skimmer voltage, 60 V; octopole DC1 and DC2 voltage, 34.4 and 34.2 V; octopole RF, 250 V.

Once the optimum culture was selected, the separation, fraction collection, and analysis of compounds present in the selected bacterial culture supernatant was performed with a HPLC/MS system. The bacterial supernatant was injected onto a C18 HPLC column. After injection, the column was washed with water/acetonitrile/formic acid for five minutes. Then compounds were separated using a linear gradient from 0% to 100% water/acetonitrile/formic acid for 30 minutes. The column was coupled to a fraction collector and 60 fractions (1 minute each) were collected and used for biological activity screening.

Biological activity of the collected lipopeptide fractions was assessed *in vitro* against *Sclerotinia sclerotiorum, Botrytis cinerea,* and *Macrophomina phaseolina.* While biological activity against individual species was observed in a number of fractions, the fractions demonstrating activity against all three pathogens were fractions 1, 24, 39, 40, 55, and 60. The lipopeptides present and excreted by CECT 8258 in the active fractions previously separated by HPLC, against *Sclerotinia sclerotiorum, Botrytis cinerea* and *Macrophomina phaseolina* are listed in the table 5 below.

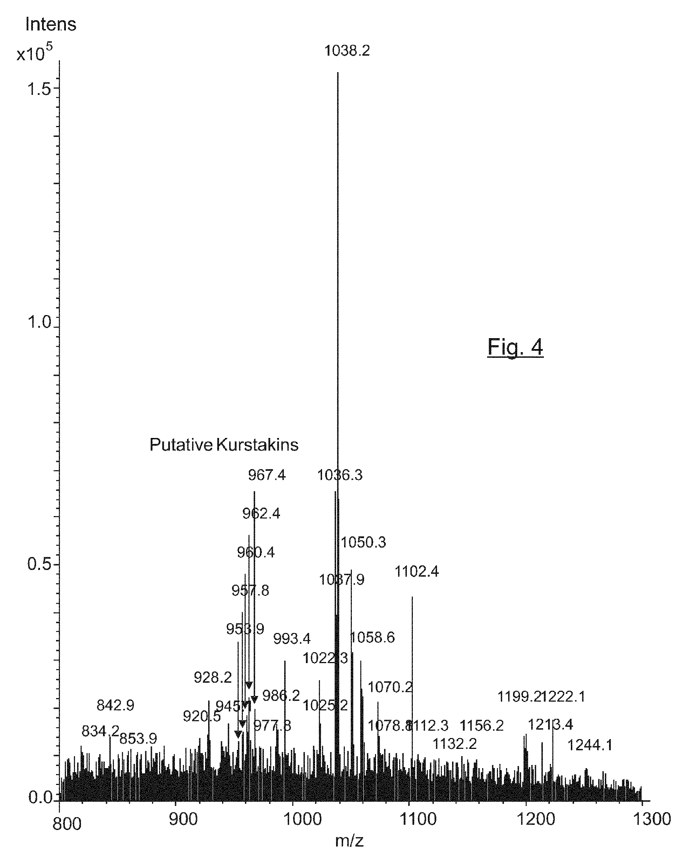

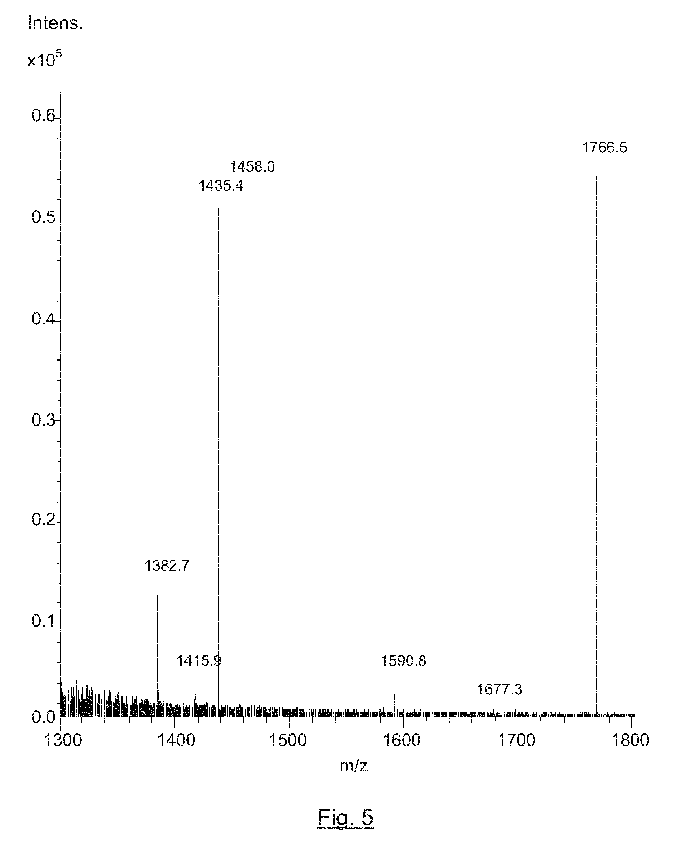

A combined MALDI-TOF mass spectrometry, LC/ Mass and amino acids analysis of these fractions were used to identify a number of lipopeptide components, including surfactin, itiurins, mycosubtilin, polymyxin, bacillomycin, putative kurstakins, and fengycin. Among these were two iturins, belonging to the iturin A family, not previously described in patents or the literature, found in fractions 1 and 39. Amino acid analysis of these novel iturins revealed the presence of cysteine and arginine. The structure of these two novel iturins are shown below. Figures 4 and 5 show the spectra of active metabolites produced by CECT 8258 from 800 to 1800 m/z.

The iturin-likes compounds were determined to be a mixture of surfactins, fengycins and a novel molecule by a combination of amino acids analysis and LC mass spectrometry (HPLC: 1 minutes, intensity of peak: 11716). New SYM I contains the following amino acids: Asn; Tyr; Asn; Gln; Prol;Asn; Ser and Cys. This make up differs from Iturin by the addition of Cys at the final chain. The molecular weight of SYM I is 1213,4 which corresponds to the following structure:

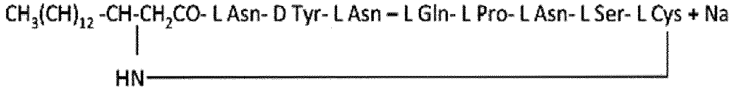

Mass spectrometry and amino acids analysis of SYM II (HPLC: 39 minutes intensity of peak: 10858) suggest that its structure is similar to SYM I with the substitution of Cys by Arg. The molecular weight of SYM II is 1199 which corresponds to the following structure:

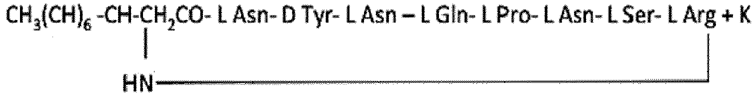

**Table 5. Lipopeptides present and excreted by Bacillus subtilis strain CECT 8258 in the active fractions previously separated by HPLC.**

| | Molecular Mass | Intensity | Name | Structure |
|---|---|---|---|---|
| Fraction 1 | 993,4 | 28706 | Su rfactin | C13 Val |
| | 1458,0 | 50325 | Fengycin | C14 Na |
| | 1213,4 | 11716 | New Iturins SYM I | Iturin+Cys |
| | | | | |
| Fraction 24 | 1025,2 | 11548 | Iturin | C10+K |
| | 1078,8 | 10805 | Mycosubtilin | C15+Na |
| | | | | |
| Fraction 39 | 928,5 | 21035 | Kurstakin | C13+Na |
| | 953,9 | 12277 | Kurstakin | C14 |
| | 1037,1 | 26116 | Iturin | C12+Na |
| | 1037,9 | 52394 | Iturin | C12+Na |
| | 1058,6 | 11638 | Iturin | C15 |
| | 1199 | 10854 | New Iturin SYM II | Iturin+Arg |
| | | | | |
| Fraction 40 | 945,1 | 16148 | Kurstakin | |
| | 986,2 | 15822 | Iturin | C10 |
| | 1038,2 | 143529 | Iturin | C12+Na |
| | 1058,7 | 23288 | Su rfactin | C15Leu+Na |
| | 1112,3 | 7729 | Bacillomycin | |
| | | | | |
| Fraction 55 | 945,3 | 6826 | Kurstakin | Kurst+K |
| | 1022,3 | 25120 | Su rfactin | C14Leu |
| | 1036,3 | 67817 | Su rfactin | C15Leu |
| | 1044,3 | 11501 | Su rfactin | C14+Na |
| | 1050,3 | 48629 | Su rfactin | C16Leu |
| | 1058,3 | 29201 | Su rfactin | C15+Na |
| | 1070,2 | 20568 | Iturin | C16 |
| | 1078,3 | 7544 | Mycosubtilin | C15+Na |
| | 1083,3 | 6378 | Iturin | C17 |
| | | | | |
| Fraction 60 | 1031,0 | 996 | Surfactin | C13Leu+Na |
| | 1011.0 | 1167 | Iturin | |
| | 1102.4 | 43922 | Iturin | |
| | 1435,4 | 51432 | Fengycin | C14 |

## Claims

1. A *Bacillus subtilis* strain deposited under deposit number CECT 8258.

2. A method for protecting or treating plants, plant parts, plant roots or plant seeds from fungal and bacterial infestations, comprising applying
- a composition comprising the *Bacillus subtilis* strain deposited under deposit number CECT 8258 or
- a supernatant obtained from a culture of the *Bacillus subtilis* strain deposited under deposited number CECT 8258 or
- an extract isolated from *Bacillus subtilis* strain deposited under deposited number CECT 8258
to said plants, plant parts, plant roots or plant seeds.

3. The method according to claim 2, **characterized in that** said fungal infestations are caused by a member of the *Sclerotineaceae* family.

4. The method according to any one of claims 2 or 3, **characterized in that** said fungal infestations are caused by fungi selected from the group composed by *Erwinia amylovora, Macrophomina phaseolina* and *Botrytis cinerea.*

5. The method according to any one of claims 2 to 4, **characterized in that** said fungal infestations are caused by foliar fungal pathogens which attack the plant foliage.

6. The method according to any one of claims 2 to 5, **characterized in that** said fungal infestations are caused by soil fungal pathogens which attack plant roots.

7. The method according to any one of claims 2 to 6, **characterized in that** a biological or chemical pesticide is further applied to said plants, plant parts, plant roots or plant seeds.

8. The method according to any one of claims 2 to 7, **characterized in that** the *Bacillus subtilis* strain deposited under deposit number CECT 8258 is applied as a whole broth culture.

9. The method according to any one of claims 2 to 8, **characterized in that** the *Bacillus subtilis* strain deposited under deposited number CECT 8258 is applied to the soil surrounding the plant roots.

10. The method according to any one of claims 2 to 9, **characterized in that** the *Bacillus subtilis* strain deposited under deposit number CECT 8258 is applied as wettable powders, granules, aqueous suspensions, emulsifiable concentrates or microencapsulations.

11. A compound of formula

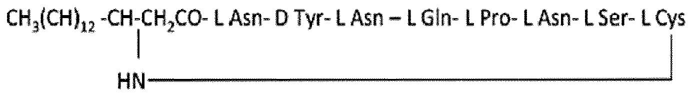

12. A method for protecting or treating plants, plant parts, plant roots or plant seeds from fungal and bacterial infestations, comprising applying the compound of formula 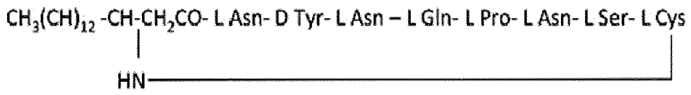 to said plants, plant parts, plant roots or plant seeds.

13. A compound of formula

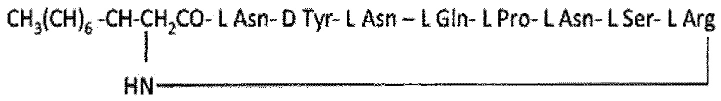

14. A method for protecting or treating plants, plant parts, plant roots or plant seeds from fungal and bacterial infestations, comprising applying the compound of formula 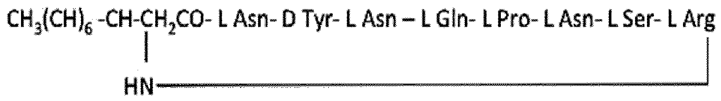 to said plants, plant parts, plant roots or plant seeds.

15. The method according to any one of claims 12 or 14, **characterized in that** said fungal infestations are caused by a member of the *Sclerotineaceae* family.

16. The method according to any one of claims 12 or claims 14 to 15, **characterized in that** said fungal infestations are caused by fungi selected from the group composed by *Erwinia amylovora, Macrophomina phaseolina* and *Botrytis cinerea.*

17. The method according to any one of claims 12 or claims 14 to 16, **characterized in that** said fungal infestations are caused by foliar fungal pathogens which attack the plant foliage.

18. The method according to any one of claims 12 or claims 14 to 17, **characterized in that** said fungal infestations are caused by soil fungal pathogens which attack plant roots.

19. The method according to any one of claims 12 or claims 14 to 18, **characterized in that** a biological or chemical pesticide is further applied to said plants, plant parts, plant roots or plant seeds.

20. The method according to any one of claims 12 or claims 14 to 19, **characterized in that** the *Bacillus subtilis* strain deposited under deposit number CECT 8258 is applied as a whole broth culture.

21. The method according to any one of claims 12 or claims 14 to 20, **characterized in that** the *Bacillus subtilis* strain deposited under deposited number CECT 8258 is applied to the soil surrounding the plant roots.

22. The method according to any one of claims 12 or claims 14 to 21, **characterized in that** the *Bacillus subtilis* strain deposited under deposit number CECT 8258 is applied as wettable powders, granules, aqueous suspensions, emulsifiable concentrates or microencapsulations.
